# EUROPEAN PATENT SPECIFICATION

(11) **EP 3 569 146 B1**
(45) Date of publication and mention of the grant of the patent: **23.09.2020**
(21) Application number: 18709056.8
(22) Date of filing: 09.01.2018
(51) Int. Cl.: A61B 5/154, A61B 5/15, B01L 3/00

(54) **CONTAINER DEVICE FOR COLLECTING, STORING AND PROCESSING BLOOD OR A BLOOD COUMPOUND**
BEHÄLTERVORRICHTUNG ZUM SAMMELN, SPEICHERN UND VERARBEITEN VON BLUT ODER EINER BLUTVERBINDUNG
DISPOSITIF FORMANT CONTENANT POUR LA COLLECTE, LE STOCKAGE ET LE TRAITEMENT DU SANG OU D'UN COMPOSÉ SANGUIN

(30) Priority: 12.01.2017 ES 201730029
(43) Date of publication of application: 20.11.2019
(73) Proprietor: Biotechnology Institute, I MAS D, S.L., 01005 Vitoria (ES)
(72) Inventor: ANITUA ALDECOA, Eduardo, 01005 VITORIA (ES)
(74) Representative: Cueto, Sénida
(86) International application number: PCT/ES2018/070012
(87) International publication number: WO 2018/130734

(56) References cited:
- EP-A1- 2 070 476
- EP-A1- 2 870 913
- DE-A1- 3 018 262
- DE-B- 1 127 031
- DE-B1- 1 818 046
- US-A- 3 931 815
- US-A- 4 890 627
- US-A- 5 086 783

## Description

### Field of the Disclosure

The disclosure relates to a container device for collecting, storing or processing blood or a blood compound. More particularly, the disclosure relates to a container device inside which a vacuum is created just before its use, and inside which blood or a blood compound is stored or processed, and from which it is possible to transfer the blood or compound to a second container, all of which can be carried out without the need to use a needle.

### Prior Art

The extraction of a small amount of blood from a human or animal patient is normally carried out using a small container and a butterfly needle. The container has an interior space in which there is a depression (commonly referred to as a "vacuum"), and a perforable end or cap. In turn, the butterfly needle consists essentially of a narrow tube ending in two needles, a first needle intended to be inserted into the patient's vein, and a second needle intended to pierce the cap of the container. When the end needles of the butterfly needle prick the vein and the container, communication is established between the vein and the interior of the container, and the blood is suctioned from the vein to the interior of the second container due to the pressure difference. The butterfly may also comprise a shutter for opening and closing the flow of fluid through the tube, enabling the practitioner to commence and finish the blood extraction by operating the shutter.

Once the blood extraction to the container has been completed, the blood is usually processed inside this container. For example, the container may be placed inside a centrifugal machine and centrifuged at a certain speed for a defined time in order to separate the blood into fractions (for example, a red cell fraction, a white cell fraction, and a fraction of platelet-rich plasma). During or after the blood processing, it is common to have to extract all or part of the contents of the container. To do so, a needle is usually inserted through the perforable cap, suctioning all or part of the contents of the container. Once the contents have been extracted, the needle is removed and the perforable cap once more seals the container. In other cases, the cap is removed and the needle or cannula is introduced into the container to suction the required fraction or fractions.

The use of needles involves certain risks. Obviously, there is the risk of the practitioner pricking him/herself with the needle. Furthermore, there is the risk of the needle being contaminated and this contamination being passed through the perforable cap and entering the container when the needle pierces the perforable cap.

On the other hand, containers used to extract blood are generally fitted with the aforementioned internal depression during production. In other words, the containers are marketed with internal "vacuum" conditions. However, over time, all plastic containers with an internal vacuum gradually lose the vacuum (i.e. there is a gradual increase in the internal pressure). Therefore, when using a plastic container with internal vacuum conditions that has been manufactured some time ago, there is a possibility that the container will not work properly, i.e., will not able to extract the necessary volume of blood. Some examples of this type of container are described in the patent documents EP2870913B1 of the same applicant, US4890627A and US3931815A.

Specifically, the first publication EP2870913B1, by the same applicant, describes a device for the collection, storage and processing of blood or other substances with the aim of solving the problem of vacuum loss over time. The device comprises a body within which a plunger moves. The body has a first end and a second end. The plunger protrudes from the second end and delimits the inside of the body airtightly. The first end preferably ends in a connector that allows the coupling of other devices. The connector is a luer-lock or similar type. The plunger comprises a shaft and a plug with a detachable and pluggable connection. The devise also comprises an internal sealant which can be pierced to seal airtightly the first end of the body when a needle is inserted. Communication from the inside to the outside will only occur when a needle is inserted to pierce the sealing element.

The patent document US4890627A, describes a cylindrical tube comprising at one end a sealing member and a piston element, which preserves the vacuum inside the tube. At the opposite end it comprises a detachable plug. A non-extendable element extends inside the tube and is connected to the piston and plug. Between the seal member and the piston is a teardrop plane at the center joint that allows the piston to separate from the seal element. In addition, the seal member and the piston comprise a needle coupling cavity, which allows communication between the inside of the tube and the fluid to be collected. The piston element comprises a conical receptacle with a narrow pitch to accommodate a conical locking pin fully connected to the non-extendable element. In this way, when the plug is pulled, the non-extensible element is tightened, transferring the force of a user to the locking pin inserted in the piston and thus separating the piston from the sealing element and generating the vacuum inside the tube due to the fact that the piston is in airtight contact with the tube walls on the inside and outside of the piston. In conclusion, the aforementioned patent document aims to achieve a vacuum tube for blood collection through the use of a needle.

The purpose of this invention is to solve at least one of the aforementioned problems, i.e., solve the problems derived from the use of needles while using a blood extraction container and/or solve the problem of the loss of internal vacuum conditions throughout the time during which the container is stored prior to its use. If possible, a container is also sought that avoids problems of contamination and manipulation of the blood and its derivatives (problems that are associated with the performance of processes in an open circuit in which the contents of the container are exposed to the atmosphere), avoiding the need to use laminar flow hoods.

### Brief Description of the Disclosure

The object of the disclosure is a container device for collecting, storing and processing blood or a blood compound. The device comprises a hollow tube, a piston set and a valve set. The tube comprises a tubular body and delimits an interior through space which ends in a proximal opening located at the proximal end of the tube and in a distal opening located at the distal end of the tube. At the distal end, the tube is fitted with a connector. The piston set, in turn, can move in the interior space of the tube and comprises a piston head and a handle, which can preferably be disconnected from the piston head and reconnected to the piston head. The piston head is positioned at a distal end of the piston set and comes into fluid-tight contact with the tubular body of the tube delimiting and isolating two regions in the interior space of the tube. The handle extends from the piston head and protrudes from the tube at the proximal end of the tube. The valve set, in turn, blocks the flow of fluid at a distal end of the interior space of the tube preventing the flow of fluid through the distal opening of the tube. The valve set is operable, such as by pressure or perforation from the exterior, to unblock the flow of fluid through the distal end of the interior space and allow the flow of fluid through said distal opening.

The device as per the disclosure allows extracting blood, storing blood, processing the extracted blood and delivering all or part of the processed blood into a container, without the need for needles. This increases the safety of the user, simplifies the execution of the process and reduces the risk of contamination of the biological substances involved. Furthermore, it allows for the extraction, storing, processing and introduction of the blood in a container to be carried out in a closed circuit, i.e. without the need to open the device or expose the blood or its derivatives to the atmosphere; this eliminates the need for a costly laminar flow installation.

### Summary of the Invention

In accordance with the present invention, there is provided a container device as defined claim 1. In addition, further advantageous embodiments follow from the dependent claims.

### Brief Description of the Figures

The details of the invention can be seen in the accompanying figures, which do not intend to limit the scope of the invention:
- Figure 1 shows a top perspective view of a tube of a device as per an illustrative embodiment of the invention.
- Figure 2 shows a bottom perspective view of the tube of Figure 1.
- Figure 3 shows a cross-sectional front elevation view of the tube of Figure 1.
- Figure 4 shows a top perspective view of a handle of a device in accordance with an illustrative embodiment of the invention.
- Figure 5 shows a bottom perspective view of the handle of Figure 4.
- Figure 6 shows a cross-sectional front elevation view of the handle of Figure 4, wherein the section has been carried out in accordance with section plane 6-6 indicated in Figure 4.
- Figure 7 shows a top perspective view of a piston head of a device in accordance with an illustrative embodiment of the invention.
- Figure 8 shows a bottom perspective view of the piston head of Figure 7.
- Figure 9 shows a front elevation view of the piston head of Figure 7.
- Figure 10 shows a cross-sectional side elevation view of the piston head of Figure 7, wherein the section has been carried out in accordance with section plane 10-10 indicated in Figure 9.
- Figure 11 shows a top perspective view of a septum of a valve set of a device in accordance with an illustrative embodiment of the invention.
- Figure 12 shows a bottom perspective view of the septum of Figure 11.
- Figure 13 shows a front elevation view of the septum of Figure 11.
- Figure 14 shows a cross-sectional side elevation view of the septum of Figure 11, wherein the section has been carried out in accordance with section plane 14-14 indicated in Figure 13.
- Figure 15 shows a top perspective view of a base of a valve set of a device in accordance with an illustrative embodiment of the invention.
- Figure 16 shows a bottom perspective view of the base of Figure 15.
- Figure 17 shows a front elevation of the base of Figure 15.
- Figure 18 shows a cross-sectional side elevation view of the base of Figure 15, wherein the section has been carried out in accordance with section plane 18-18 indicated in Figure 17.
- Figure 19 shows a top perspective view of a hood of a device in accordance with an illustrative embodiment of the invention.
- Figure 20 shows a bottom perspective view of the hood of Figure 19.
- Figure 21 shows a front elevation of the hood of Figure 19.
- Figure 22 shows a cross-sectional side elevation view of the hood of Figure 19, wherein the section has been carried out in accordance with section plane 22-22 indicated in Figure 21.
- Figure 23 shows a cross-sectional front elevation of the assembled device with the handle in an advanced position in accordance with an illustrative embodiment of the invention.
- Figure 24 shows an enlarged view of the distal end of the device of Figure 23.
- Figure 25 shows six steps of an illustrative sequence of use of the device of Figure 23.
- Figure 26 shows four remaining steps of said illustrative sequence of use of the device of Figure 23.
- Figure 27 shows an enlarged view of the distal end of the device in the position of the fifth step of Figure 25.
- Figure 28 shows an enlarged view of the proximal end of the tube, with the piston head clipped to this proximal end.
- Figure 29 shows an enlarged view of the distal end of the device in the position of the third step of Figure 26.
- Figure 30 shows an enlarged view of the upper part of a device in accordance with a second illustrative embodiment of the invention.
- Figure 31 shows seven steps of another illustrative sequence of use of the device of Figure 23 without the hood.
- Figure 32 shows six steps of yet another illustrative sequence of use of the device of Figure 23 without the hood.
- Figure 33 shows five subsequent steps following those of Figure 32.
- Figure 34 shows seven steps of another illustrative sequence of use of the device of Figure 23 without the hood.
- Figure 35 shows seven subsequent steps following those of Figure 34.
- Figure 36 shows seven steps of yet another illustrative sequence of use of the device of Figure 23 without the hood.
- Figure 37 shows seven subsequent steps following those of Figure 36.
- Figure 38 shows eight steps of yet another illustrative sequence of use of the device of Figure 23 without the hood.

### Brief Description of the Figures

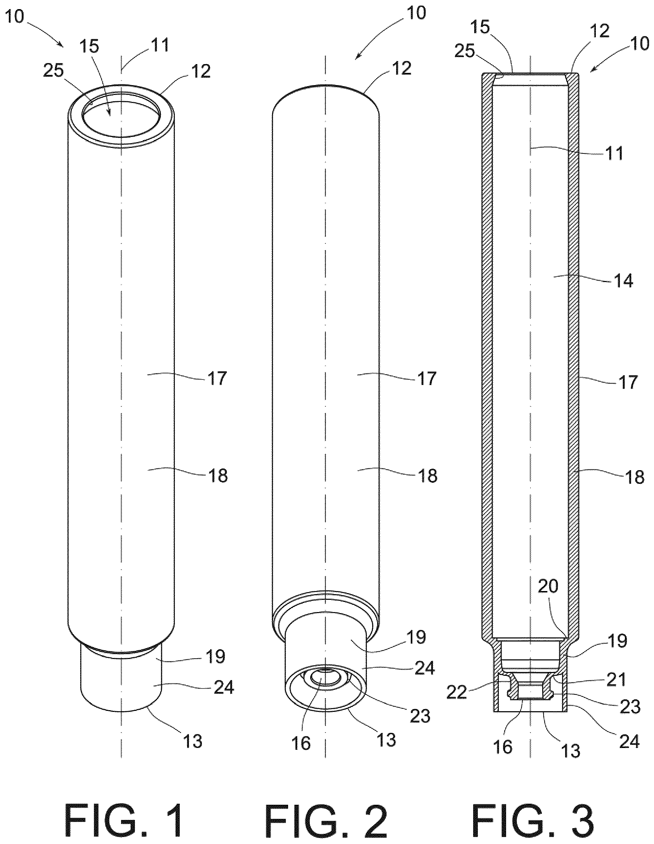

Figures 1 to 22 show the different parts or components of a container device (1) in accordance with an illustrative embodiment of the invention. This device can be used to collect, store or process blood or a blood compound, as will be explained in greater detail herein.

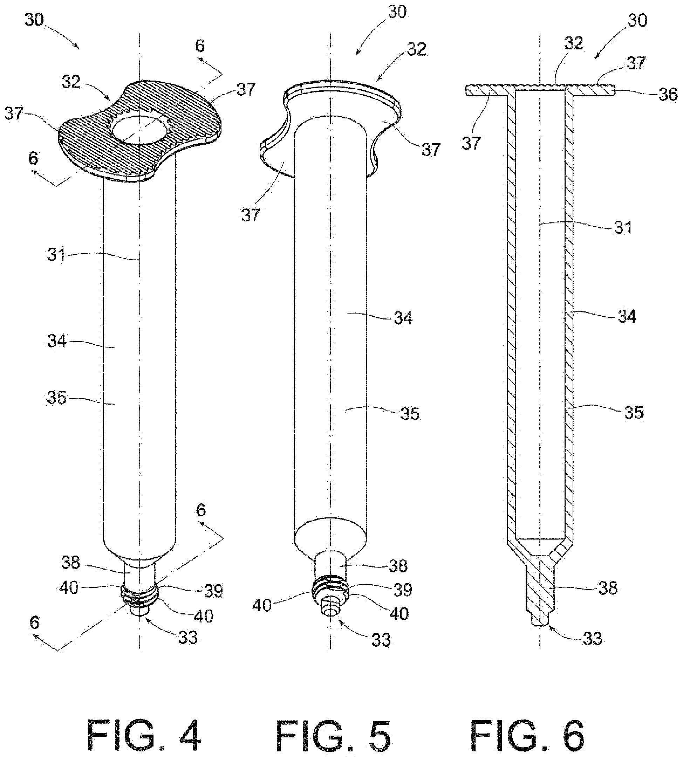

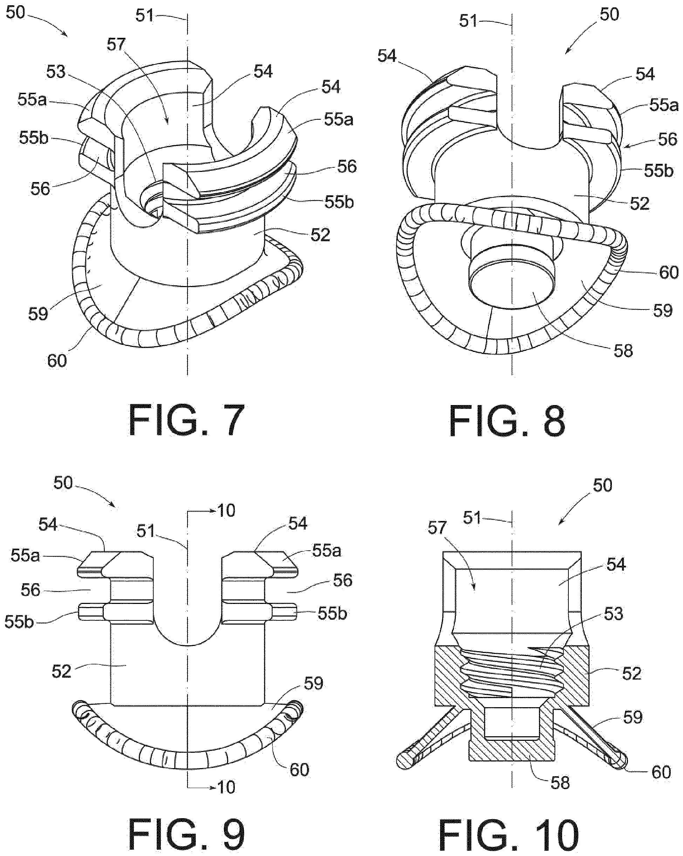

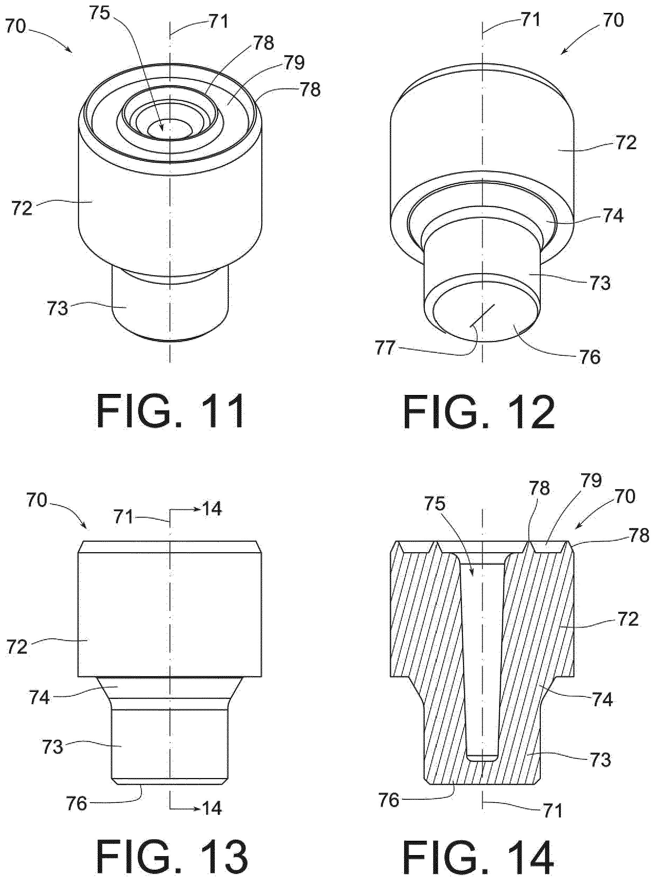

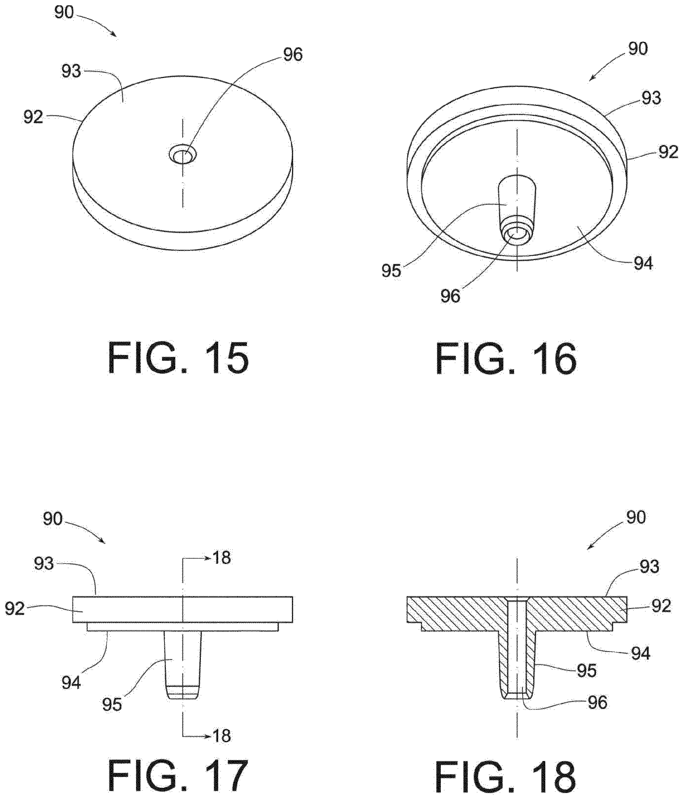

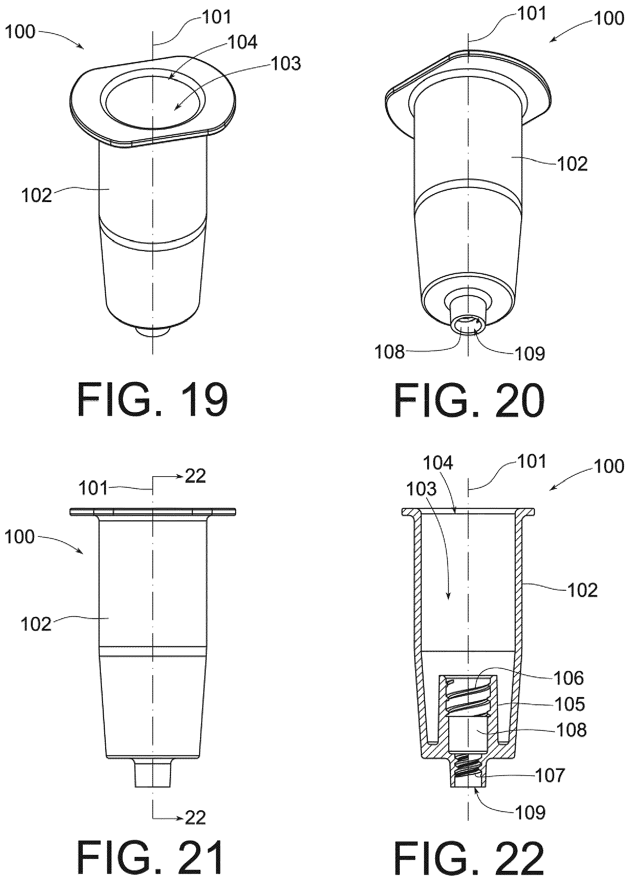

The container device (1) of the present example comprises a tube (10), shown in Figures 1 to 3, inside which a piston set (30, 50) moves. The piston set (30, 50) is made up of a piston shaft or handle (30), shown in Figures 4-6, and a piston head (50), shown in Figures 7-10. As will be seen in greater detail herein, a distal end (13) of the tube (10) is closed by a deformable and perforable valve set (70, 90), which is made up of a septum (70) and a base (90), shown respectively in Figures 11-14 and in Figures 15-18. The device (1) may also comprise a hood (100), shown in Figures 19-22.

Figures 1-3 show the tube (10) of the device (1) in detail. The tube (10) is formed along a longitudinal axis (11) and has a proximal end (12) and a distal end (13). The tube (10) is hollow, presenting an interior space (14) that extends along the whole length of the tube (10) from the proximal end (12) to the distal end (13) and ends in a proximal opening (15) and in a distal opening (16). The tube (10) is essentially comprised of a tubular body (17) formed by a cylindrical wall (18) which surrounds the interior space (14). Following the wall (18), there is a second cylindrical wall (19) which has a smaller diameter than the wall (18) and also surrounds the interior space (14). Between this wall (18) and the second wall (19) there is a transition shoulder (20). After the second wall (19) and behind a second transition shoulder (21) there is a third essentially cylindrical wall (22) that is fitted with a connector (23). In some embodiments, such as the one shown in the figures, the connector (23) is a threaded connector. This connector (23) is preferably able to connect to a syringe; for example, the connector (23) can be a threaded male "luer-lock" termination, of the type defined in the ISO 594, DIN/EN 1707:1996 and 20594-1:1993 standards. A generally cylindrical, encircling wall or skirt (24) extends distally from the second wall (19), surrounding the third wall (22) and the connector (23). The skirt (24) may be built as a continuation of the second wall (19), i.e. having the same shape as the second wall (19). The function of the skirt (24) is to protect the connector (23) from any contact with other bodies during the handling of the device (1), for which it is preferable for the skirt (24) to be longer than the connector (23), i.e. it preferably protrudes distally with respect to the connector (23), such as in the illustrated embodiment. Finally, at the proximal end (12) of the tube (10) there is a lip (25) which fully surrounds the proximal opening (15) and causes the proximal opening (15) to have a smaller diameter than the inner diameter of the tubular body (17).

Figures 4-6, in turn, show the piston axis or handle (30). This handle (30) is formed around a longitudinal axis (31) and has a proximal end (32) and a distal end (33). The handle (30) is essentially comprised of a tubular body (34) formed by a substantially cylindrical wall (35). At the proximal end (32) of the handle (30), the handle (30) is fitted with a grip or gripping area (36), in this case in the form of two flat parts (37) that extend transversally with respect to the longitudinal axis (31) and protrude towards the sides of the tubular body (34) in order to help the user pull or push the handle (30) in the direction of the longitudinal axis (31), as will be seen herein. At the distal end (33) of the handle (30) there is a neck (38) that is narrower (i.e. provided with a smaller width or diameter) than the tubular body (34). A connector (39), such as a male threaded termination, is provided on the neck (38) for disconnectably connecting the piston head (50), as will be seen in greater detail herein. The connector (39) of the present embodiment is a male threaded termination with threads that do not extend along a full 360-degrees turn about the longitudinal axis (31), but rather extend along two opposed, threaded lugs (40), which is why this threaded termination cannot be seen in the transversal section in Figure 6.

Figures 7-10, in turn, illustrate the piston head (50). This piston head (50) is formed along a longitudinal axis (51) and comprises a body (52) having an internal connector (53). In the present case, the connector (53) is a female, threaded connector. Both the body (52) and the connector (53) are formed along the longitudinal axis (51). Two protrusions (54), which are slightly elastic (i.e. have a tendency to recover their rest position, shown in the figure), extend proximally from a proximal end of the body (52) and at opposite sides of the body (52). Two transversal protrusions (55) extend radially outwards from each protrusion (54). The two transversal protrusions (55) are slightly separated from each other so that a space (56) is provided therebetween, wherein the space (56) is oriented radially or transversally outwards. The piston head (50) comprises an interior cavity (57) which extends between the two protrusions (54) and through the connector (53). This interior cavity (57) is blind, i.e. closed at the distal end by a wall (58). The piston head (50) further comprises a skirt (59) which extends distally and flares outward from the body (52) and, in the present embodiment, has an undulated edge. This edge (60), as will be seen in detail hereinafter, is configured to come into contact with the wall (18) of the tubular body (17) of the tube (10) along the whole perimeter of the edge (60) in order to fully seal the piston head (50) against said wall (18).

Figures 11-14 show several views of the septum (70) of the valve set (70, 90). The septum (70) is formed along a longitudinal axis (71) and comprises a first portion (72) and a second portion (73) that is narrower than and extends distally from the first portion (72). In the present embodiment, the first portion (72) and the second portion (73) are cylindrical and are connected by a conical part (74) or portion having a decreasing width. The septum (70) has an interior cavity (75) which extends from the proximal end of the first portion (72), through the first portion (72) and the second portion (73), and is closed at its distal end by a wall (76). As can be seen, the interior cavity (75) may be slightly conical. As can be observed in Figure 12, a slit or cut (77) may extend through the wall (76). The cut (77) is normally closed, preventing the flow of fluid through the wall (76). The wall (76) may be deformed in such a way that, when the wall (76) is deformed, the cut (77) opens, allowing the flow of fluid to and from the interior cavity (75). Alternatively or additionally to the cut (77), the wall (76) may be perforable to allow the insertion of a needle through the wall (76) and into the interior cavity (75). At the proximal end, the septum (70) is provided with circular ribs or protrusions (78) that are slightly flexible and positioned in the direction of the longitudinal axis (71). Spaces (79) are defined between the ribs or protrusions (78).

Figures 15 to 18 show the base (90) of the valve set (70, 90). The base (90) is formed along a central longitudinal axis (91) and comprises a disc (92) arranged concentrically to the central longitudinal axis (91). The disc (92) has a proximal side (93) and a distal side (94). A neck (95) extends distally from the distal side (94) of the disc (92). This neck (95) is slightly conical in order to be press fitted inside the interior cavity (75) of the septum (70). The base (90) further comprises a longitudinal, through interior cavity (96), which extends completely through the base (90) in the direction of the longitudinal axis (91). This interior cavity (96) is arranged around the longitudinal axis (91).

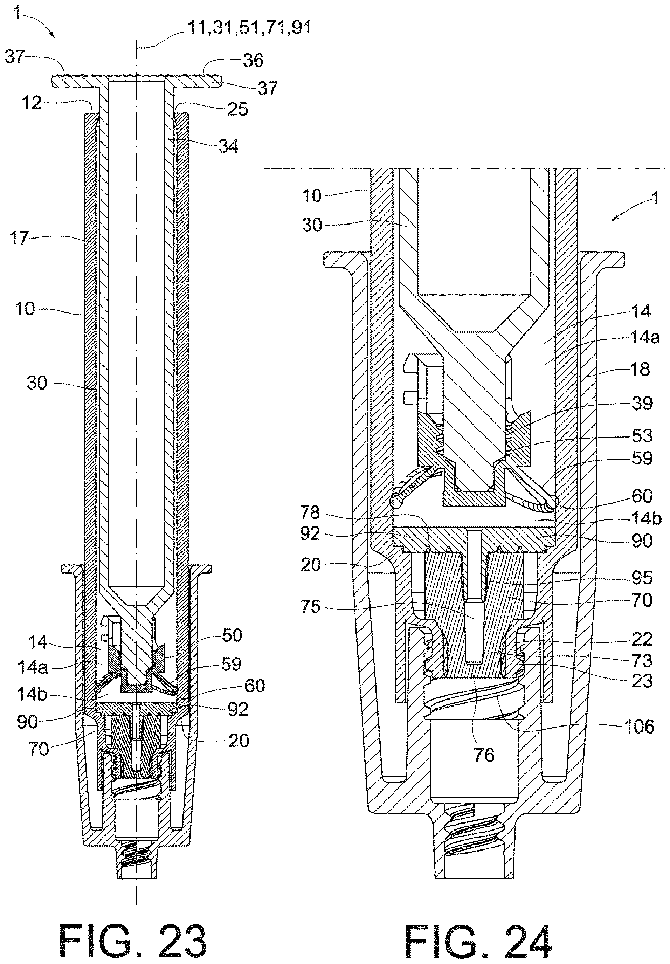

Finally, Figures 19 to 22 show the hood (100), which is arranged along a longitudinal axis (101) and comprises a hollow main body (102) having an interior space (103) sized to receive the tubular body (17) of the tube (10) of Figures 1 to 3. At a proximal end of the hood (100), there is an opening (104) to allow for the insertion of the tube (10) in the interior space (103). At a distal end of the hood (100), a neck (105) extends towards the interior space (103). This neck (105) has a first connector, which in this case is a first threaded connection (106), for the connection of the connector (23) of the tube (10). The first threaded connection (106) of this embodiment is female, and preferably a threaded female "luer-lock" termination, similar to that defined in the ISO 594, DIN/EN 1707:1996 and 20594-1:1993 standards. Furthermore, a second threaded connection (107) is positioned distally with respect to the first threaded connection (106). This second threaded connection (107) is preferably a female threaded connection configured to be connected to a male threaded connection (126) of a butterfly needle (120), as shown in Figure 24. An interior duct (108) extends through the neck (105), the first threaded connection (106) and the second threaded connection (107) and up to a distal opening (109).

Figures 23 and 24 show the device (1) in an assembled state. As can be seen, the valve set (70, 90) is placed inside the tube (10), in the interior space (14). The second portion (73) of the septum (70) of the valve set (70, 90) is in the interior space (14), surrounded by -and preferably adjusted against- the third wall (22) of the tube (10), so that the septum (70) remains affixed to this third wall (22) by friction. In turn, the base (90) is coupled to the septum (70) by having the neck (95) of the base (90) introduced and retained by friction within the interior cavity (75) of the septum (70). The septum (70) is coupled to the base (90) with a slight pressure towards the base (90) such that the circular protrusions (78) of the septum (70) are in fluid-tight contact with the distal side (94) of the base (90), producing a seal that guarantees fluid-tightness with the base (90). The disc (92) of the base (90) is supported on the shoulder (20) of the tube (10).

The handle (30) is inserted inside the tube (10), in the interior space (14) of the tube (10). The connector (39) (in this case, a threaded termination) of the handle (30) is connected to the connector (53) (in this case, a threaded connector) of the piston head (50) in such a way that it can be disconnected (in this case by unthreading). As the handle (30) and the piston head (50) are connected, they can move together or in unison along the interior space (14) of the tube (10). The proximal end of the handle (30) protrudes from the proximal end (12) of the tube (10), such that the gripping area (36) remains outside the tube (10) and is accessible for the user to push or pull the handle (30). The lip (25) of the proximal end (12) of the tube (10) adjusts against the tubular body (34) of the handle (30).

In the situation in Figures 23 and 24, the edge (60) of the skirt (59) of the piston head (50) is in fluid-tight contact with the wall (18) of the tubular body (17) of the tube (10) along the whole perimeter of the edge (60), providing an absolute sealing of the piston head (50) against this wall (18) and separating the interior space (14) of the tube (10) into two chambers or regions (14a, 14b). The region (14b) located distally (in front) of the piston head (50) is sealed or fluid-tightly closed by the piston head (50), by the wall (18) of the tube (10) and by the valve set (70, 90), and more particularly by the wall (76) of the septum (70) of the valve set (70, 90) which closes the interior cavity (75) of the septum (70).

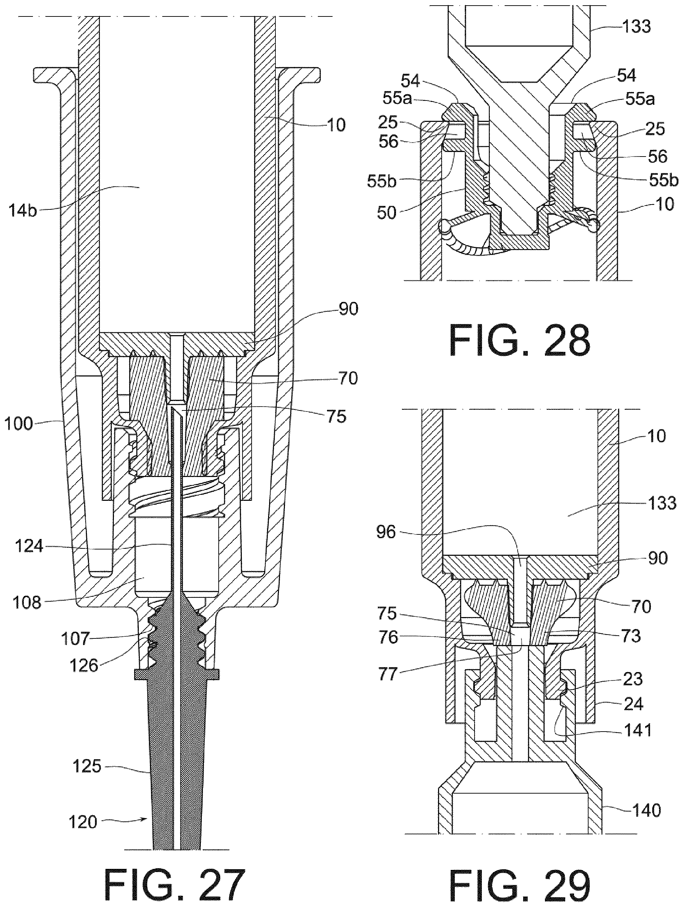

In the situation in Figures 23 and 24, the handle (30) is in an advanced position with respect to the tube (10), i.e. inserted as far as possible or almost as far as possible inside the tube (10). If the user proceeds to pull the handle (30) rearwards, exerting an appropriate force against the flat parts (37) of the handle (30) to attempt extract it from the tube (10), the handle (30) begins to move proximally, increasing the volume of the region (14b) located in front of the piston head (50) and reducing the volume of the region (14a) located behind the piston head (50). The air from the region (14a) can pass between the lip (25) and the tubular body (34) and exit towards the exterior of the tube (10), allowing the handle (30) to move rearward; this air that exits the interior space (14) of the tube (10) between the lip (25) and the tubular body (34) of the handle (30) prevents the entry of bacteria and contaminating agents towards the interior space (14) of the tube (10). In turn, the increase in the volume of the fluid-tight region (14b) causes a depression in this region (14b). By making the handle (30) move sufficiently rearward, there comes a moment, illustrated in Figure 28, in which the rear protrusion (55a) of the piston head (50) moves beyond the lip (25) of the tube (10) and the lip (25) is received in the space (56) between the protrusions (55a, 55b) causing the piston head (50) to become clipped to the proximal end (12) of the tube (10).

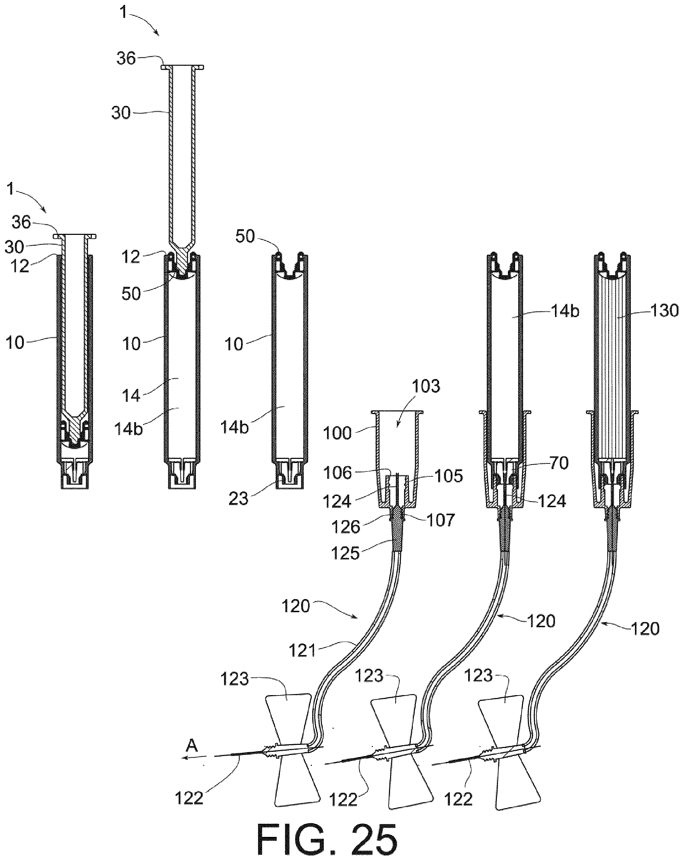

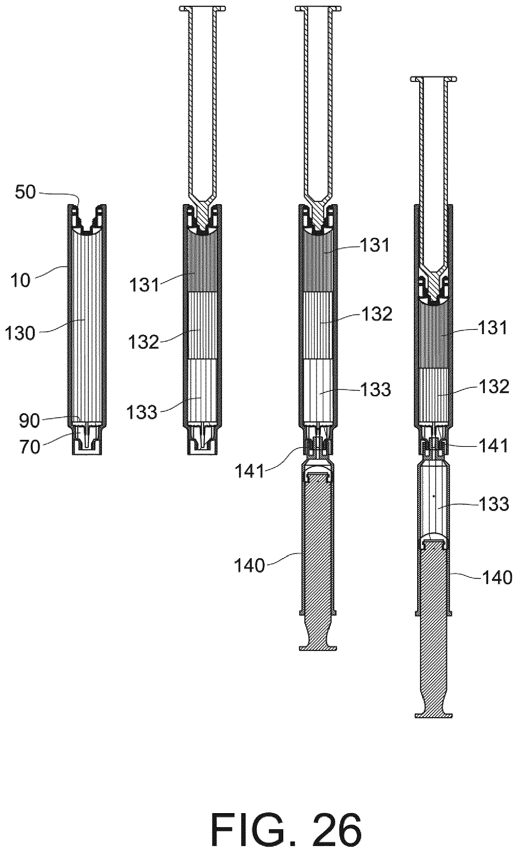

Figures 25 and 26 show a sequence of steps as per an example of use of the aforementioned device (1), where Figure 25 shows six steps and Figure 26 illustrates four steps of the sequence.

As can be seen in Figure 25, the sequence of use begins with the device (1) in the situation of Figure 23, i.e. with the handle (30) in an advanced position inside the tube (10). Then, in a second step, the user pulls the gripping area (36) of the handle (30) and causes the handle (30) to displace rearward inside the tube (10) until the piston head (50) is clipped to the proximal end (12) of the tube (10) (as previously described with reference to Figure 28) and a depression has been formed in the region (14b). In a third step, the user turns the handle (30) with respect to the longitudinal axis (31), unthreading the connector (39) of the handle (30) from the connector (53) of the piston head (50), and proceeds to remove the handle (30), leaving the device (1) in a configuration in which it resembles a conventional vacuum tube, of the type that is used to remove relatively small amounts of blood from a human or animal body. Then, in a fourth step of the sequence, a butterfly needle (120), of the type known in the art, is provided. The butterfly needle (120) comprises a flexible tube (121), at one end of which there is a first needle (122) accompanied by a flat part or a butterfly (123), and at the opposite end of which there is a second needle (124) which extends from a connector (125) that has a male threaded connection (126). In this fourth step of the sequence, the first needle (122) is inserted into a patient's vein, pressing the butterfly (123) against the patient's skin to stabilize the first needle (122), and the second needle (124) is inserted in the interior duct (108) of the hood (100) whilst the threaded connection (126) of the connector (125) is threaded into the second threaded connection (107) of the hood (100). Then, in the fifth step of Figure 25 (illustrated in greater detail in the enlarged view in Figure 27) the device (1), which has interior vacuum conditions (i.e. which has a depression in the region (14b)) is inserted into the interior space (103) of the hood (100) and the connector (23) of the tube (10) is threaded to the first threaded connection (106) of the hood (100), causing the second needle (124) of the butterfly needle (120) to either perforate the wall (76) of the septum (70) of the valve set (70, 90) or to pass through the cut (77). In consequence, the second needle (124) becomes communicated with the interior cavity (75) of the septum (70) and with the interior cavity (96) of the base (90). In this way, a fluid passageway is established between the patient's vein and the region (14b) and, due to the pressure difference, blood starts flowing from the vein towards the region (14b). Eventually, the situation in the final step of Figure 25, in which the region (14b) has been filled with blood (130), is reached.

In a further step (first step of Figure 26), the user unthreads and disconnects the butterfly needle (120) from the tube (10), thereby obtaining a closed tube (10) that is filled with blood (130) and is ready to be processed. For example, processing may consist in centrifuging the tube in a centrifugal machine to separate the blood (130) into two or more fractions (131, 132, 133) as is well known in the prior art of medicine and odontology. Once the blood is separated into fractions (131, 132, 133), the user proceeds with the second step of Figure 26, which consists in reconnecting the handle (30) to the piston head (50) by threading the connector (39) of the handle (30) to the connector (53) of the piston head (50). Then, as shown in the third step of Figure 26, the user proceeds to connect a conventional syringe (140), of the type which comprises a female "luer-lock" threaded connection (141) at its distal end, by threading this female "luer-lock" threaded connection (141) to the male threaded "luer-lock" connector (23) of the tube (10). As can be seen in the enlarged view of Figure 29, in connecting the threaded connection (141) to the tube (10), the threaded connection (141) pushes the septum (70) of the valve set (70, 90) inwards, causing the compression of the second portion (73) and causing the cut (77) to open forming fluid passageway for the fluid to pass through the wall (76) of the septum (70), thus leaving the interior of the syringe (140) in communication with the region (14b) of the device (1) through the open cut (77), the interior cavity (75) and the interior cavity (96). Then, as shown in the final step of Figure 26, the user pushes the handle (30) with a certain force, releasing the clipping between the protrusions (55a, 55b) of the piston head (50) and the lip (25) of the tube (10). It could also happen that the clipping between the protrusions (55a, 55b) of the piston head (50) and the lip (25) of the tube (10) has already been released due to the centrifugal force during the centrifugation process. By continuing to push the handle (30), the handle (30) and the piston head (50) move forward along the interior space (14) of the tube (10), pushing the desired fraction or fractions (133, 132, 131) so that they transfer partially or entirely to the syringe (140) (for example, in the illustrated method, only the full front fraction (133) is transferred). Once the desired fraction or fractions (133, 132, 131) have been transferred, the user can unthread the syringe (140) from the device (1) and use the fraction or fractions (133, 132, 131) (in this case, the fraction (133)) contained in the syringe (140) for diverse medical applications. Note that in other examples of use of the device (1), a same syringe (140) can receive the contents of more than one device (1).

In summary, the device (1) as per the invention allows blood to be extracted, the extracted blood to be processed and all or part of the processed blood to be introduced into a conventional syringe, without requiring the use of needles or exposing the contents of the device to the exterior. This increases the safety of the user, simplifies the performance of the process and reduces the risk of contamination of the biological substances involved.

As an alternative to what is illustrated in the sequence of Figures 25 to 26, a conventional hood of the type that does not have a first threaded connection (105) can be used instead of the hood (100). In this case, the hood would be fixed by pressure or friction to the skirt (24) of the tube (10) and/or to the surface of the tubular body (17) of the tube (10). I.e., the hood (100) described in this document is optional and the device (1) can be used with conventional blood extraction hoods. However, the hood (100) described in this document is advantageous because it allows the exact depth of insertion of the needle to be selected. Furthermore, by turning the first threaded connection (106) of the hood (100) with respect to the tube (10), the valve set (70, 90) can be gradually opened, allowing to regulate the flow of the fluid passing through the valve set (70, 90).

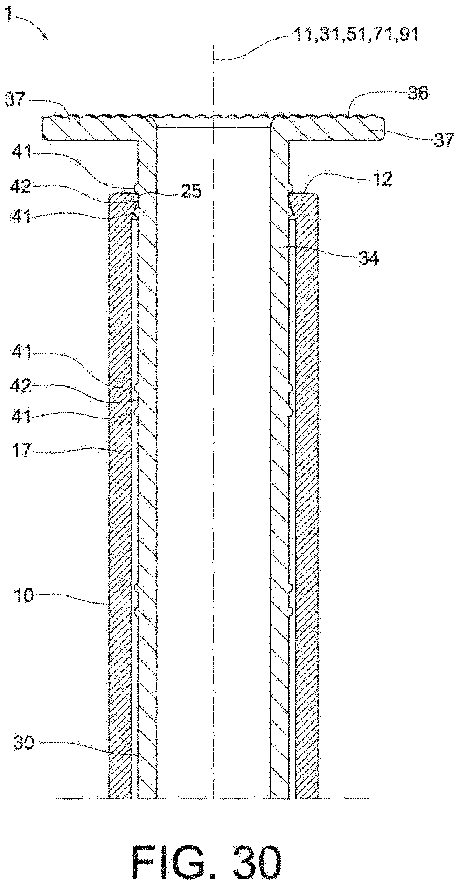

Figure 30 shows an alternative embodiment of the invention, in which the tubular body (34) of the handle (30) comprises some protuberances (41) protruding radially from the tubular body (34). As can be seen, there is a retention valley or space (42) between two consecutive protuberances (41), wherein the space (42) is preferably sized such that the lip (25) is retained and relatively snugly fit between these two consecutive protuberances (41) and within the space (42), retaining the handle (30) so that it does not move longitudinally unless the user pulls or pushes the handle (30) with a force greater than a predetermined threshold. As can be seen in the figure, the protuberances (41) are preferably organised in pairs, the handle (30) having at least two pairs of protuberances (41). Between each pair of protuberances (41) there is a separation that is larger than the length of the space (42). In this way, the handle (30) provides at least two retention spaces (42) for the lip (25). The user can pull the handle (30) and, as the handle (30) is removed from the tube (10), the lip (25) moves along the tubular body (34) and is retained in each space (42), whilst the device makes a slight clicking sound whenever the lip (25) exceeds a protuberance (41) and enters inside the space (42). In short, this embodiment allows the handle (30) to be adjusted in different discrete positions with respect to the tube (10), whereby each position corresponds to a specific volume of the vacuum-provided chamber (14b). The adjustment is highly intuitive thanks to the successive audible clicks being emitted whenever a discreet position is reached.

Finally, in alternative embodiments of the invention, the piston head (50) and the handle (30) may be formed into a single piece, which can be manufactured for instance by injection moulding.

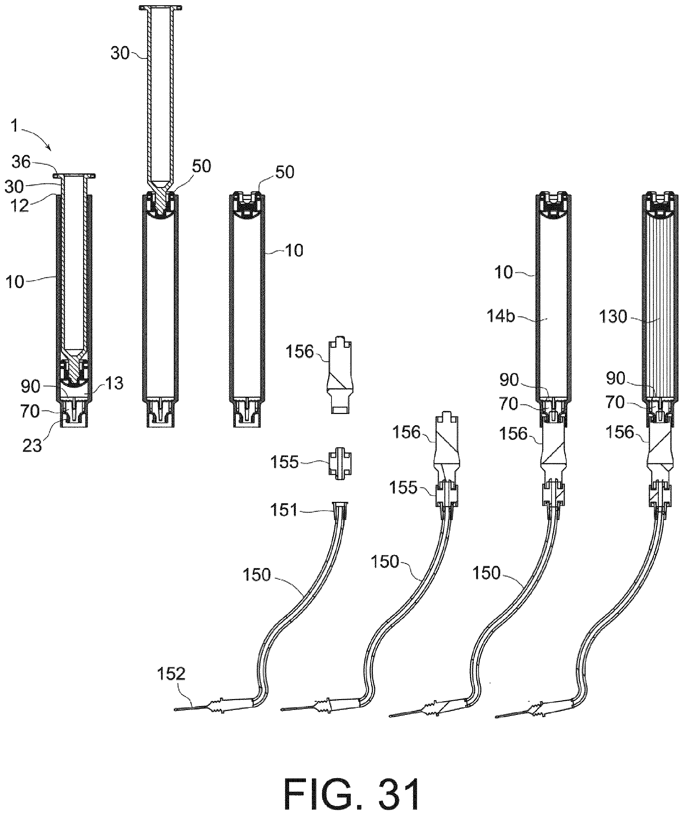

Figure 31 shows a sequence of steps in accordance with a further illustrative use of the device (1). The first three steps of the sequence are the same as the first three steps of Figure 25 and allow to configure the device (1) to have an internal depression or vacuum, a fluid-tight closure on the proximal end (12) and a valve set (70, 90) arranged blocking the flow of fluid at a distal end (13). Next, in a fourth step of the sequence, a butterfly needle (150) is obtained, having a female termination (151), a male-male connector (155) and a male-female valve (156), all of which are commercially available products. In a fifth step of the sequence, one end of the male-male connector (155) is connected to the male-female valve (156) and the other end is connected to the butterfly needle (150). Then, in a sixth step of the sequence, the first needle (152) of the butterfly needle (150) is inserted in a patient's vein and the connector (23) of the tube (10) is connected to the male-female valve (156) which is connected to the butterfly needle (150). The latter causes the valve set (70, 90) and the valve (156) to open and, in consequence, a fluid passageway to form between the patient's vein and the region (14b) of the tube (10), flow starting to flow towards the region (14b) due to pressure difference. Eventually, the situation of the seventh step of Figure 31, in which the region (14b) has filled with blood (13), is reached. The procedure may then continue with the steps of Figure 26, which have been described heretofore.

Thus, in the sequence of Figure 31, a valve (156) having a female-male luer-lock connection (which is commercial) and a male-male connector (which is also commercial) are used instead of the hood (100) of Figures 19-22 and 25. Replacing the hood (100) with the valve (156) leads to an increase in fluid-tightness within the system. The blood remains within the butterfly needle (150) isolated from the outside. When the valve (156) of the butterfly needle (150) is connected to the valve set (70, 90) of the tube (10), the blood enters the tube (10) without having had a direct contact with the outside.

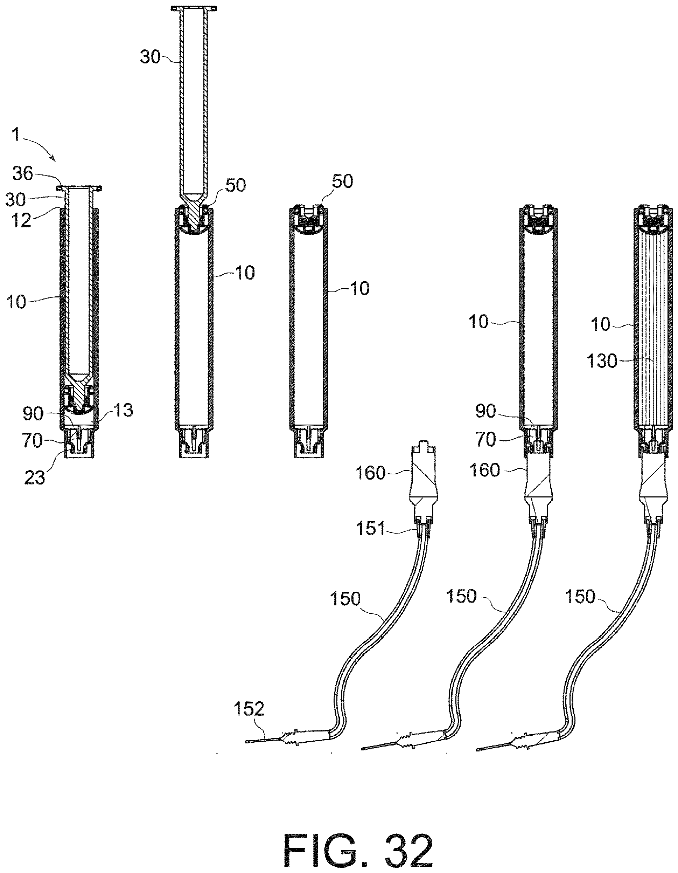

Figure 32 shows a sequence of steps in accordance with a further use of the device (1). This sequence is almost identical to the sequence of Figure 31, except for the fact that the butterfly needle (150) is provided with a male-male valve (160), i.e. a male connection capable of directly connecting to the butterfly needle (150) without the need for an adapter. Having the valve (160) connect directly to the butterfly needle (150) is advantageous in that, in this particular location, the system is subjected to no contamination as the inside of the butterfly needle (150) is isolated and the fluid passageway towards the tube (10) is opened only when the two valves (160; 70, 90) are connected to one another (fifth step of Figure 32), thereby allowing the blood to remain isolated from the outside at all times.

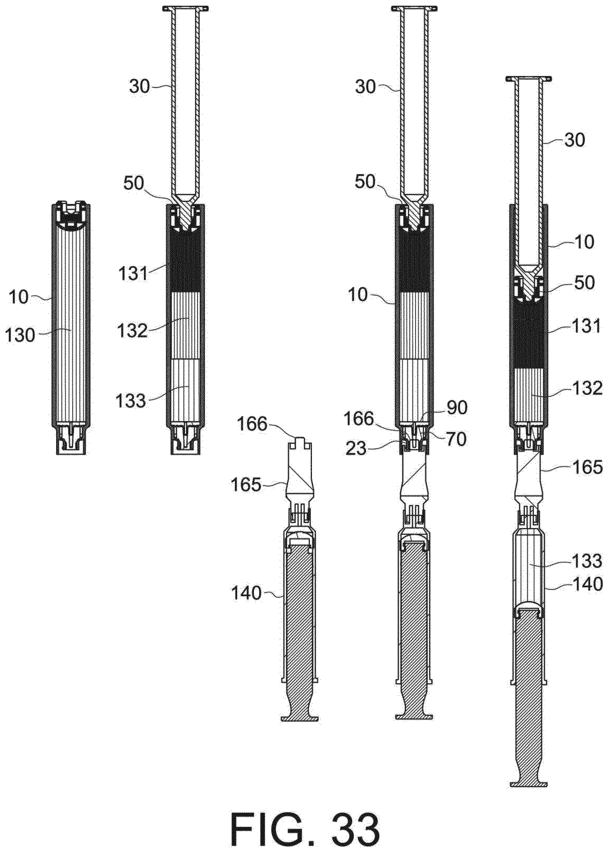

Similarly to the method of Figure 31, the method of Figure 32 can be followed by the steps of Figure 26, which have been described heretofore. Alternatively, in another example of use of the device in accordance with the invention, the process of Figure 32 may be followed by the steps of Figure 33. In other words, after extracting blood as shown in Figure 32, once the tube (10) filled with blood is obtained (first step of Figure 33), the tube (10) is centrifuged to separate the blood in two or more fractions (131, 132, 133). Once the blood has been separated into fractions (131, 132, 133), the user proceeds with the second step of Figure 33, which consists in re-connecting the handle (30) to the piston head (50). Next, as shown in the third step of Figure 33, the user takes a commercial syringe (140) provided with a valve (165) having a female-male luer-lock connection (i.e. providing a male luer-lock connection (166) it its distal end) to increase fluid-tightness. Next, as shown in the fourth step, the male luer-lock connection (166) of the valve (165) of the syringe (140) is inserted in the threaded male "luer-lock" connector (23) of the tube (10), and the male luer-lock connection (166) pushes the septum (70) of the valve set (70, 90) inwards, opening the valve set (70, 90) and causing the formation of a fluid passageway through the valve set (70, 90). Similarly, connecting the valve (165) to the tube (10) causes the valve (165) to open and allow fluid to pass therethrough. Next, as shown in the last step of Figure 33, the user pushes the handle (30) with a certain force causing the clipping between the piston head (50) and the tube (10) to end, if not previously undipped, and the handle (30) to move forward pushing the desired fraction or fractions (133, 132, 132), partially or in their entirety, to cause a transfer thereof into the syringe (140) for further medical use.

In other words, the steps of Figure 33 are similar to those of Figure 26, with the exception that a female-male valve (165) is added to the fractioning syringe (140). This valve (165) can come incorporated to the syringe (140) and packaged with the syringe (140), in which case the interior of the syringe (140) remains sterilized even after opening the packaging. Furthermore, the transfer is carried out with no communication with the outside and thus preventing any kind of dripping when the syringe (140) is disconnected from the tube (10). Thus, by combining the valve (160) of the extraction butterfly needle (150) (Figure 32) with the valve (165) of the fractioning needle (140) (Figure 33), the blood and plasma are never exposed to the outside, thus allowing the system to be not only closed but also hermetic.

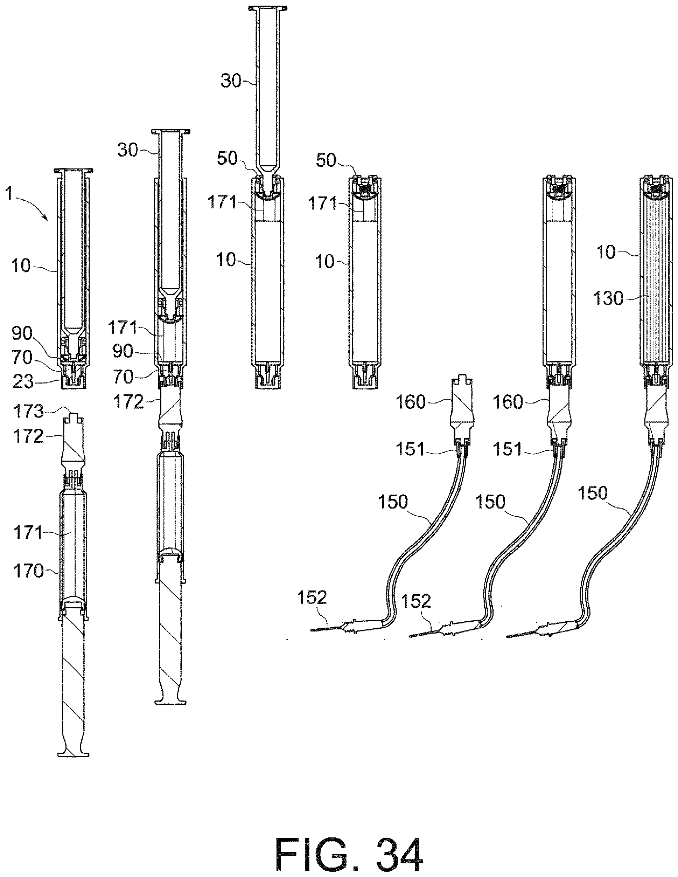

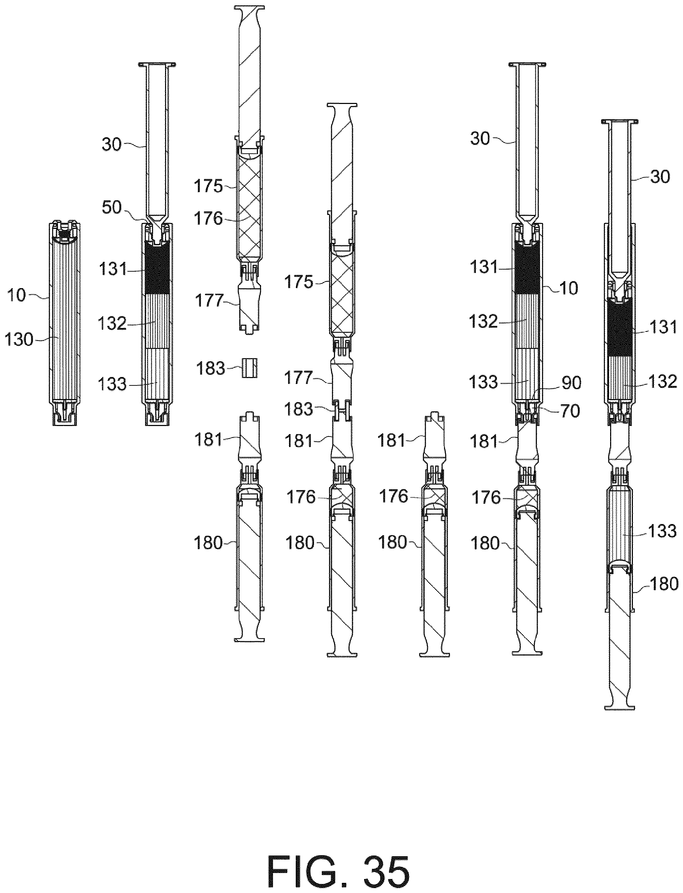

Figures 34 and 35 show a sequence of steps in accordance with a further use of the device (1). In a first step of the sequence, the process begins with the compressed tube (10), and an anticoagulant (e.g., sodium citrate) is added to the tube (10). For this purpose, as shown in the first step of Figure 34, a syringe (170) previously loaded with a certain amount of anticoagulant (171) and having a valve (172) with a male luer-lock connection (173) is used. As shown in the second step, the syringe (170) is connected to the tube (10), thereby opening the valve set (70, 90) and the valve (172), and the required dose of anticoagulant (171) is transferred to the tube (10). The syringe (170) is then removed, and the valve set (70, 90) of the tube (10) automatically shuts closed. Next, as shown in the third step, the handle (30) is pulled to cause a vacuum. When the handle (30) reaches the end of its displacement, the handle (30) becomes clipped to the proximal end (12) of the tube (10). Then, in the fourth step, the handle (30) is removed. Next, blood is hermetically extracted using a butterfly needle (150) as explained with reference to Figure 32, thereby obtaining a closed, fluid-tight tube (10) containing blood (130) as shown in the first step of Figure 35. Next, the tube (10) is centrifuged and the handle (30) is reconnected to the piston head (50), as shown in the second step of Figure 35. In a third step, a first needle (175), preloaded with an activating substance (176) (e.g., calcium chloride) and with a female-male valve (177), is arranged facing a second needle (180), the purpose of which is to receive one or more fractions (131, 132, 133) and which has a female-male valve (181). A female-female connector (183) is disposed between the syringes (175, 180). The valves (177, 181) maintain fluid-tightness inside the respective syringes (175, 180). The transfer takes place in a sterile space and thus the female-female connector (183) is sterile. Next, the first syringe (175), which is preloaded with the activating substance (176), is connected to the fractioning, second syringe (180) and the required dose of activating sequence (176) is transferred to the second syringe (180), as shown in the fourth step. In a fifth step, the first syringe (175), its valve (177) and the female-female connector (183) are disconnected. In a sixth step, the second syringe (180) is connected to the tube (10), and the valve (181) presses and opens the valve set (70, 90) of the tube (10). In a seventh step, fractioning is performed in the second syringe (180) by pressing the handle (30) of the tube (10) until the desired fraction or fractions (131, 132, 133) are delivered to the second syringe (180).

In other words, the method of Figures 34 and 35 is the same as the method of Figures 32 and 33, with the exception that sodium citrate and calcium chloride are added using preloaded syringes which come with luer connection valves. This can be considered a hermetic circuit, as there is no way its contents can become contaminated; the blood, plasma and surfaces contacted thereby are never exposed to the outside during the manipulation procedure.

In an alternative embodiment, it would be possible to add the additives of the first step of Figure 34 and the fourth step of Figure 35 from an ampoule or other container, using a syringe.

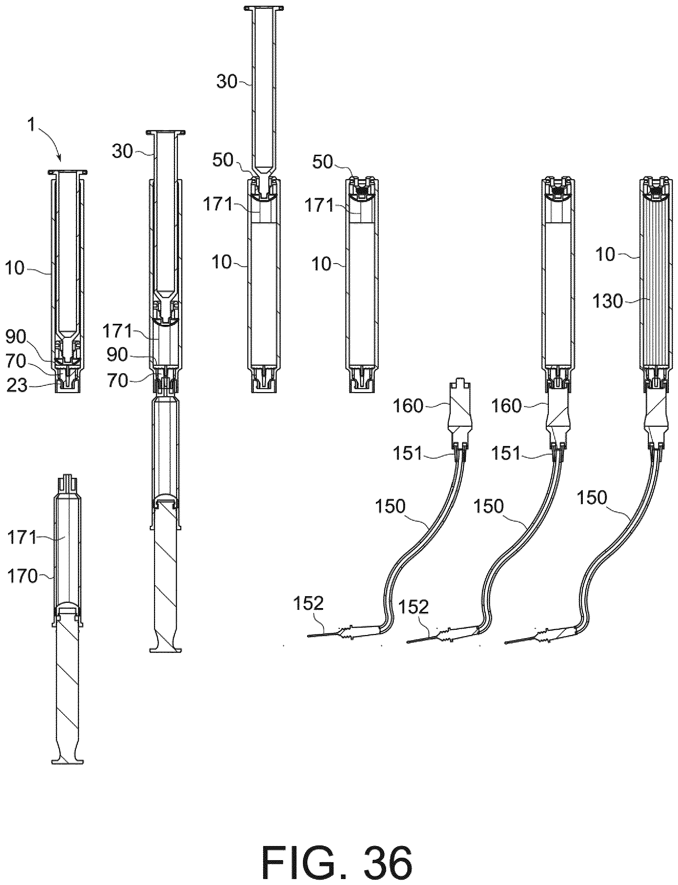

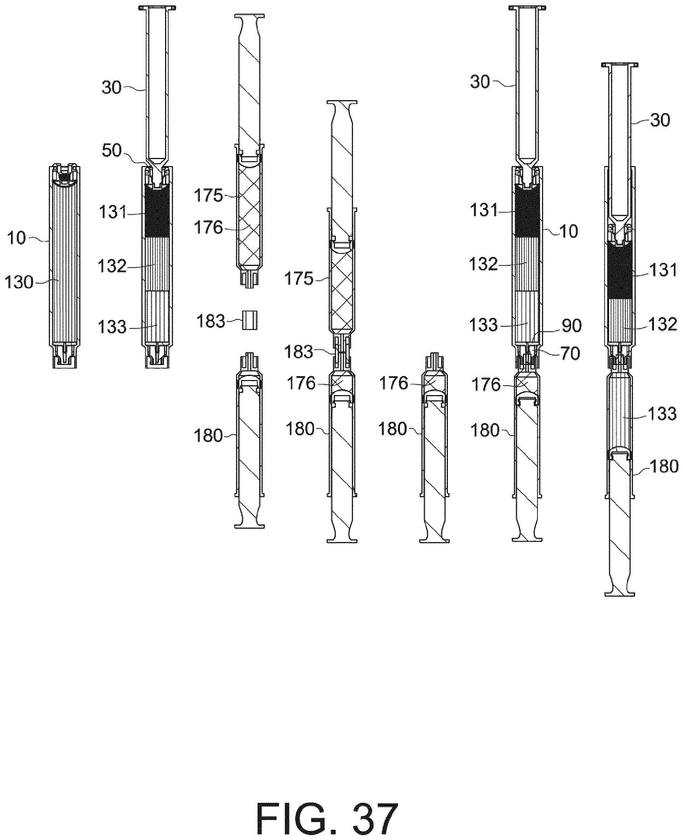

Figures 36 and 37 show a sequence of steps in accordance with a further use of the device (1). The sequence is similar to the sequence of Figures 34 and 35, but does not use the most compromised valves, i.e. the valve (172) which connects to the syringe (170) containing anticoagulant (171) (Figure 34), the valve (177) which connects to the first syringe (175) (Figure 35) and the valve (181) which connects to the fractioning or second syringe (180) (Figure 35). However, the valve set (70, 90) of the tube (10) remains, keeping the contents of the tube (10) protected.

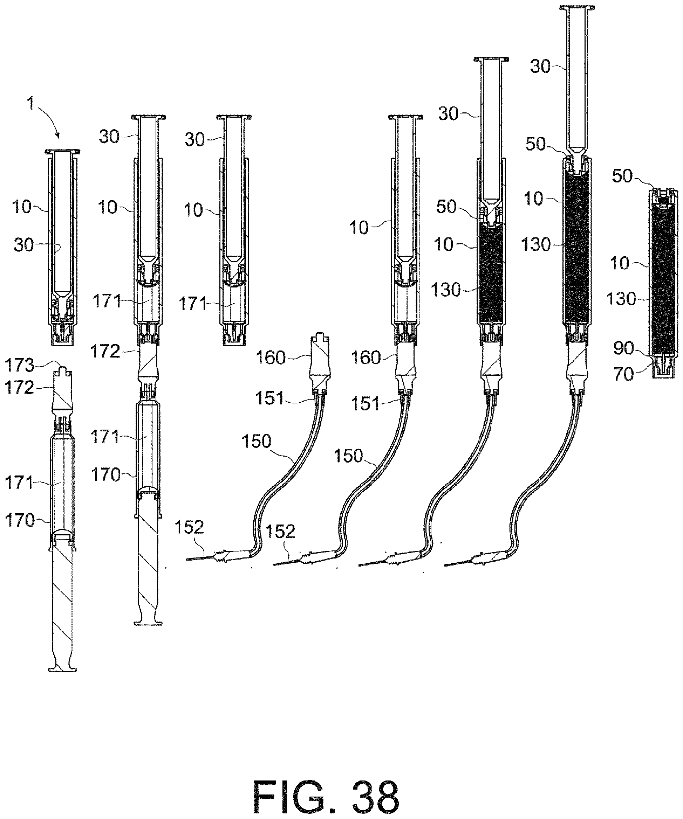

Figure 38 shows a sequence of steps in accordance with another example of use of the device (1). In this sequence, the tube (10) is operated like a syringe while extracting blood. In a first step, the syringe (170) preloaded with anticoagulant (171) (e.g., sodium citrate) and provided with a valve (172) is obtained, together with the tube (10) in accordance with the invention, arranged in its forwardmost position. In a second step, the syringe (170) with anticoagulant (171) is connected to the tube (10) and the required dose of anticoagulant (171) is transferred to the tube (10). Next, as shown in the third step, the tube (10) is disconnected from the preloaded syringe (170). In a fourth step, a butterfly needle (150) having a valve (160) with a male outlet is obtained, and the needle (152) of the butterfly needle (150) is inserted into the patient's vein. In a fifth step, the tube (10) is connected to the valve (160) which is in turn connected to the butterfly needle (150). In a sixth step, the handle (30) of the tube (10) is slowly pulled so that the blood (130) starts flowing into the tube (10) by suction. The handle (30) continues to be pulled until the piston head (50) reaches the end of its trajectory and becomes clipped to the proximal end of the tube (10), as shown in the seventh step in the figure (note that it would be possible to pull the handle (30) without clipping the piston head (50) and without completely filling the tube (10), which could be of interest in some medical applications). Next, the handle (30) is removed, thereby obtaining a tube (10) filled with blood (130), with the tube (10) fluid-tightly closed by a valve set (70, 90), as shown in the eighth step. The procedure may then continue, for instance, in the same way as other examples described heretofore.

This use of the tube (10), by which blood is suctioned from the vein into the tube (10), allows to better control the manner in which blood is inserted, allowing to prevent haemolysis.

Methods of use of the device (1) not being part of the invention are also contemplated which are similar to the aforementioned methods but in which blood is extracted from an IV instead of using a butterfly needle (150).

## Claims

1. Container device (1) for collecting, storing and processing blood or a blood compound wherein the container device (1) comprises, a hollow tube (10) and a piston set (30, 50), where the hollow tube (10) comprises a tubular body (17) and delimits an interior space (14) that ends in a proximal opening (15) located in a proximal end (12) of the tube (10) and in a distal opening (16) located in a distal end (13) of the tube (10), wherein the tube (10) further comprises a connector (23) at the distal end (13), and where the piston set (30, 50) is movably arranged in the interior space (14) of the tube (10) and which comprises a piston head (50) and a handle (30), wherein the piston head (50) is arranged at a distal end of the piston set (30, 50) and contacts the tubular body (17) of the tube (10) delimiting and isolating two regions (14a, 14b) in the interior space (14) of the tube (10), wherein the handle (30) extends from the piston head (50) and protrudes from the tube (10) at the proximal end (12) of the tube (10), wherein the container device (1) is **characterised in that** it comprises:
- a valve set (70, 90) positioned so as to block the flow of fluid in a distal end of the interior space (14) of the tube (10) and prevent the flow of fluid through the distal opening (16) of the tube (10), wherein the valve set (70, 90) is operable by a pressure exerted on a distal wall (76) of the valve set (70, 90) to unblock the flow of fluid through the distal end of the interior space (14) and allow the flow of fluid through the distal opening (16), wherein the wall (76) comprises a through cut (77) that adopts a closed and sealed configuration in the absence of the pressure and an open configuration in the presence of the pressure.

2. Device (1), according to claim 1, **characterised in that** the valve set (70, 90) is operable by a pressure exerted on a distal wall (76) of the valve set (70, 90) towards the proximal end (12) of the tube (10), wherein the pressure produces a deformation of the valve set (70, 90) which causes the formation of a fluid passageway fluid through the valve set (70, 90).

3. Device (1), according to claim 1, **characterised in that** the valve set (70, 90) is operable by a perforation made by a needle through the valve set (70, 90), wherein the perforation forms a fluid passageway through the valve set (70, 90) and the needle.

4. Device (1), according to claim 1, **characterised in that** the handle (30) is disconnectable from and reconnectable to the piston head (50).

5. Device (1), according to claim 1, **characterised in that** the handle (30) is connected to the piston head (50) by a threaded union between a threaded connector (39) of the handle (30) and a threaded connector (53) of the piston head (50).

6. Device (1), according to claim 1, **characterised in that** the piston head (50) is clippable to the proximal end (12) of the tube (10).

7. Device (1), according to claim 6, **characterised in that** the piston head (50) comprises one or more elastically and radially deformable protrusions (54), which are clippable to the proximal end (12) of the tube (10) to retain the piston head (50) in a position adjacent to the proximal end (12) of the tube (10).

8. Device (1), according to claim 1, **characterised in that** the handle (30) has a tubular body (34) that is in contact with a lip (25) of the tube (10) on the whole of the perimeter of the tubular body (34) during the displacement of the handle (30) with respect to the tube (10).

9. Device (1), according to claim 8, **characterised in that** the handle (30) comprises at least two spaces (42) where the lip (25) can be alternatively housed to define discrete positions of the handle (30) with respect to the tube (10), whereby each discrete position corresponds to a different volume from a region (14b) of the device (1) provided with vacuum conditions.

10. Device (1), according to claim 1, **characterised in that** the connector (23) of the tube (10) is a luer connection.

11. Device (1), according to claim 10, **characterised in that** the connector (23) of the tube (10) is a male luer connection.

12. Device (1), according to claim 1, **characterised in that** it further comprises a hood (100) which has an interior space (103) sized to receive the tubular body (17) of the tube (10), and **in that** it further comprises a neck (105) that extends from a distal end of the hood (100) towards the interior space (103), wherein the neck (105) comprises a threaded connection (106) for the connection of the connector (23) of the tube (10).

13. Device (1), according to claim 12, **characterised in that** the threaded connection (106) of the hood (100) is a luer connection.

14. Device (1), according to claim 12, **characterised in that** the threaded connection (106) of the hood (100) is a female luer connection.

15. Device (1), according to claim 1, comprising a hood coupled by friction to the distal end (13) of the tube (10).

16. Device (1), according to claim 1, wherein the tube at the distal end is provided with a skirt (24) which surrounds the connector (23) and protects the connector (23).

## Patentansprüche

1. Behältervorrichtung (1) zum Sammeln, Speichern und Verarbeiten von Blut oder einer Blutverbindung, wobei die Behältervorrichtung (1) eine hohle Röhre (10) und einen Kolbensatz (30, 50) umfasst, und die hohle Röhre (10) einen Rohrkörper (17) umfasst, der einen Innenraum (14) begrenzt, welcher in einer proximalen Öffnung (15) an einem proximalen Ende (12) der Röhre (10) und in einer distalen Öffnung (16) an einem distalen Ende (13) der Röhre (10) endet, wobei die Röhre (10) ferner ein Verbindungsstück (23) an dem distalen Ende (13) umfasst, und der Kolbensatz (30, 50) beweglich im Innenraum (14) der Röhre (10) angeordnet ist, und die einen Kolbenboden (50) und einen Griff (30) umfasst, wobei der Kolbenboden (50) an einem distalen Ende des Kolbensatzes (30, 50) angeordnet ist und den Rohrkörper (17) der Röhre (10) berührt, wodurch zwei Bereiche (14a, 14b) im Innenraum (14) der Röhre (10) begrenzt und abgegrenzt werden, wobei der Griff (30) sich aus dem Kolbenboden (50) erstreckt und von der Röhre (10) an dem proximalen Ende (12) der Röhre (10) herausragt, und die Behältervorrichtung (1) **dadurch gekennzeichnet ist, dass** sie Folgendes umfasst:
- einen Ventilsatz (70, 90), so angeordnet, dass er den Flüssigkeitsstrom an einem distalen Ende des Innenraums (14) der Röhre (10) blockiert und den Flüssigkeitsstrom durch die distale Öffnung (16) der Röhre (10) verhindert, wobei der Ventilsatz (70, 90) durch einen auf eine distale Wand (76) des Ventilsatzes (70, 90) ausgeübten Druck betreibbar ist, um den Flüssigkeitsstrom durch das distale Ende des Innenraums (14) freizugeben und den Flüssigkeitsstrom durch die distale Öffnung (16) zu ermöglichen, dabei enthält die Wand (76) einen Schnitt (77), der ohne Druck eine geschlossene und abgedichtete Konfiguration und mit Druck eine offene Konfiguration annimmt.

2. Behältervorrichtung (1), in Übereinstimmung mit Anspruch 1, **dadurch gekennzeichnet, dass** der Ventilsatz (70, 90) durch einen Druck betreibbar ist, der auf eine distale Wand (76) des Ventilsatzes (70, 90) auf das proximale Ende (12) der Röhre (10) ausgeübt wird, wobei der Druck eine Deformation des Ventilsatzes (70, 90) hervorruft, was die Bildung eines Flüssigkeitsdurchgangs durch den Ventilsatz (70, 90) verursacht.

3. Behältervorrichtung (1), in Übereinstimmung mit Anspruch 1, **dadurch gekennzeichnet, dass** der Ventilsatz (70, 90) durch eine Perforation betreibbar ist, die mit einer Nadel durch den Ventilsatz (70, 90) erzeugt wird, wobei die Perforation einen Flüssigkeitsdurchgang durch den Ventilsatz (70, 90) und die Nadel bildet.

4. Behältervorrichtung (1), in Übereinstimmung mit Anspruch 1, **dadurch gekennzeichnet, dass** der Griff (30) abtrennbar von und wieder aufsetzbar auf den Kolbenboden (50) ist.

5. Behältervorrichtung (1), in Übereinstimmung mit Anspruch 1, **dadurch gekennzeichnet, dass** der Griff (30) mit dem Kolbenboden (50) durch eine Gewindeverbindung zwischen einem Gewindeanschluss (39) des Griff (30) und einem Gewindeanschluss (53) des Kolbenbodens (50) verbunden ist.

6. Behältervorrichtung (1), in Übereinstimmung mit Anspruch 1, **dadurch gekennzeichnet, dass** der Kolbenboden (50) an das proximale Ende (12) der Röhre (10) anklemmbar ist.

7. Behältervorrichtung (1), in Übereinstimmung mit Anspruch 7, **dadurch gekennzeichnet, dass** der Kolbenboden (50) eine oder mehrere elastisch und radial verformbare Ausbuchtungen (54) umfasst, die an das proximale Ende (12) der Röhre (10) anklemmbar sind, um den Kolbenboden (50) in einer an dem proximalen Ende (12) der Röhre (10) anliegenden Position zu halten.

8. Behältervorrichtung (1), in Übereinstimmung mit Anspruch 1, **dadurch gekennzeichnet, dass** der Griff (30) einen Rohrkörper (34) hat, der während der Verschiebung des Griff (30) gegenüber der Röhre (10) entlang des ganzen Umfangs des Rohrkörpers (34) in Kontakt mit einer Lippe (25) der Röhre (10) steht.

9. Behältervorrichtung (1), in Übereinstimmung mit Anspruch 8, **dadurch gekennzeichnet, dass** der Griff (30) mindestens zwei Lücken (42) umfasst, wobei die Lippe (25) wahlweise untergebracht werden kann, um unterschiedliche Positionen des Griff (30) gegenüber der Röhre (10) abzustecken, wobei jede unterschiedliche Position einem unterschiedlichen Volumen eines Bereichs (14b) der Vorrichtung (1) unter Vakuumbedingungen entspricht.

10. Behältervorrichtung (1), in Übereinstimmung mit Anspruch 1, **dadurch gekennzeichnet, dass** das Verbindungsstück (23) der Röhre (10) ein Luer-Anschluss ist.

11. Behältervorrichtung (1), in Übereinstimmung mit Anspruch 10, **dadurch gekennzeichnet, dass** das Verbindungsstück (23) der Röhre (10) ein männlicher Luer-Anschluss ist.

12. Behältervorrichtung (1), in Übereinstimmung mit Anspruch 1, **dadurch gekennzeichnet, dass** sie ferner eine Haube (100) umfasst, die einen Innenraum (103) zur Aufnahme des Rohrkörpers (17) der Röhre (10) hat, und dadurch, dass sie ferner einen Zapfen (105) umfasst, der sich von einem distalen Ende der Haube (100) zum Innenraum (103) hin erstreckt, wobei der Zapfen (105) eine Gewindeverbindung (106) für den Anschluss des Verbindungsstücks (23) der Röhre (10) umfasst.

13. Behältervorrichtung (1), in Übereinstimmung mit Anspruch 12, **dadurch gekennzeichnet, dass** die Gewindeverbindung (106) der Haube (100) ein Luer-Anschluss ist.

14. Behältervorrichtung (1), in Übereinstimmung mit Anspruch 12, **dadurch gekennzeichnet, dass** die Gewindeverbindung (106) der Haube (100) ein weiblicher Luer-Anschluss ist.

15. Behältervorrichtung (1), in Übereinstimmung mit Anspruch 1, die eine Haube umfasst, welche durch Reibung an das distale Ende (13) der Röhre (10) angekoppelt wird.

16. Behältervorrichtung (1), in Übereinstimmung mit Anspruch 1, wobei die Röhre an dem distalen Ende mit einer Schürze (24) ausgestattet ist, die das Verbindungsstück (23) umsäumt und das Verbindungsstück (23) schützt.

## Revendications

1. Dispositif de conteneur (1) pour la collecte, le stockage et le traitement du sang ou d'un composé sanguin, dans lequel le dispositif de conteneur (1) comprend un tube creux (10) et un ensemble de piston (30, 50), où le tube creux (10) comprend un corps tubulaire (17) et délimite un espace intérieur (14) qui se termine par une ouverture proximale (15) située dans une extrémité proximale (12) du tube (10) et par une ouverture distale (16) située dans une extrémité distale (13) du tube (10), dans lequel le tube (10) comprend en outre un connecteur (23) à l'extrémité distale (13), et où le ensemble de piston (30), 50) est disposé de manière mobile dans l'espace intérieur (14) du tube (10) et qui comprend une tête de piston (50) et une poignée (30), dans lequel la tête de piston (50) est disposée à une extrémité distale du ensemble de piston (30, 50) et entre en contact avec le corps tubulaire (17) du tube (10) en délimitant et en isolant deux zones (14a, 14b) dans l'espace intérieur (14) du tube (10), dans lequel la poignée (30) s'étend à partir de la tête de piston (50) et fait saillie du tube (10) à l'extrémité proximale (12) du tube (10), dans lequel le dispositif de conteneur (1) est **caractérisé en ce qu'**il comprend :
- un ensemble de soupape (70, 90) positionné de manière à bloquer l'écoulement de fluide dans une extrémité distale de l'espace intérieur (14) du tube (10) et à empêcher l'écoulement de fluide à travers l'ouverture distale (16) du tube (10), dans lequel le ensemble de soupape (70, 90) peut être actionné par une pression exercée sur une paroi distale (76) du ensemble de soupape (70, 90) pour débloquer l'écoulement de fluide par l'extrémité distale de l'espace intérieur (14) et permettre l'écoulement de fluide par l'ouverture distale (16), dans lequel la paroi (76) comprend une découpe traversant (77) qui adopte une configuration fermée et étanche en l'absence de pression et une configuration ouverte en présence de la pression.

2. Dispositif (1), selon la revendication 1, **caractérisé en ce que** le ensemble de soupape (70, 90) peut être actionné par une pression exercée sur une paroi distale (76) du ensemble de soupape (70, 90) vers l'extrémité proximale (12) du tube (10), dans lequel la pression produit une déformation du ensemble de soupape (70, 90) qui provoque la formation d'un passage de fluide à travers le ensemble de soupape (70),

3. Dispositif (1), selon la revendication 1, **caractérisé en ce que** le ensemble de soupape (70, 90) peut être actionné par une perforation réalisée par un pointeau à travers le ensemble de soupape (70, 90), dans lequel la perforation forme un passage de fluide à travers le ensemble de soupape (70, 90) et le pointeau.

4. Dispositif (1), selon la revendication 1, **caractérisé en ce que** la poignée (30) peut être déconnectée et reconnectée à la tête du piston (50)

5. Dispositif (1), selon la revendication 1, **caractérisé en ce que** la poignée (30) est reliée à la tête de piston (50) par un raccord fileté entre un connecteur fileté (39) de la poignée (30) et un connecteur fileté (53) de la tête de piston (50).

6. Dispositif (1), selon la revendication 1, **caractérisé en ce que** la tête de piston (50) peut être clipsée à l'extrémité proximale (12) du tube (10).

7. Dispositif (1) selon la revendication 6, **caractérisé en ce que** la tête de piston (50) comprend une ou plusieurs saillies (54) élastiquement et radialement déformables, qui peuvent être clipsées à l'extrémité proximale (12) du tube (10) pour retenir la tête de piston (50) dans une position adjacente à l'extrémité proximale (12) du tube (10).

8. Dispositif (1), selon la revendication 1, **caractérisé en ce que** la poignée (30) comporte un corps tubulaire (34) qui est en contact avec une lèvre (25) du tube (10) sur tout le périmètre du corps tubulaire (34) pendant le déplacement de la poignée (30) par rapport au tube (10).

9. Dispositif (1), selon la revendication 8, **caractérisé en ce que** la poignée (30) comprend au moins deux espaces (42) où la lèvre (25) peut être logée alternativement pour définir des positions discrètes de la poignée (30) par rapport au tube (10), chaque position discrète correspondant à un volume différent d'une région (14b) du dispositif (1) pourvue de conditions de vide.

10. Dispositif (1), selon la revendication 1, **caractérisé en ce que** le connecteur (23) du tube (10) est un raccord Luer.

11. Dispositif (1), selon la revendication 10, **caractérisé en ce que** le connecteur (23) du tube (10) est un raccord Luer mâle.

12. Dispositif (1), selon la revendication 1, **caractérisé en ce qu'**il comprend en outre un capot (100) qui a un espace intérieur (103) dimensionné pour recevoir le corps tubulaire (17) du tube (10), et **en ce qu'**il comprend en outre un col (105) qui s'étend d'une extrémité distale du capot (100) vers l'espace intérieur (103), dans lequel le col (105) comprend un raccord fileté (106) pour le raccordement du connecteur (23) du tube (10).

13. Dispositif (1), selon la revendication 12, **caractérisé en ce que** le raccord fileté (106) du capot (100) est un raccord Luer.

14. Dispositif (1), selon la revendication 12, **caractérisé en ce que** le raccord fileté (106) du capot (100) est un raccord Luer femelle.

15. Dispositif (1), selon la revendication 1, comprenant un capuchon couplé par friction à l'extrémité distale (13) du tube (10).

16. Dispositif (1), selon la revendication 1, dans lequel le tube à l'extrémité distale est muni d'une jupe (24) qui entoure le connecteur (23) et protège le connecteur (23).
